(19) Europäisches Patentamt
European Patent Office
Office européen des brevets

(11) Veröffentlichungsnummer: 0 239 927
A1

(12) EUROPÄISCHE PATENTANMELDUNG

(21) Anmeldenummer: 87104430.1

(22) Anmeldetag: 25.03.87

(51) Int. Cl.[4]: C07D 498/06, C07D 513/06, A01N 43/90, //(C07D498/06,265:00,209:00),(C07D498/06,265:00,221:00),(C07D513/06,279:00,209:00),(C07D513/06,279:00,221:00)

(30) Priorität: 04.04.86 JP 76625/86

(43) Veröffentlichungstag der Anmeldung: 07.10.87 Patentblatt 87/41

(84) Benannte Vertragsstaaten: CH DE FR GB IT LI

(71) Anmelder: NIHON TOKUSHU NOYAKU SEIZO K.K.
Itohpia Nihonbashi Honcho Building 7-1,
Nihonbashi Honcho 2-chome
Chuo-ku Tokyo 103(JP)

(72) Erfinder: Kurahashi, Yoshio
47-15, Oya-machi
Hachioji-shi Tokyo(JP)
Erfinder: Kagabu, Shinzo
1768-532, Sagiyama
Gifu-shi Gifu-ken(JP)
Erfinder: Matsumoto, Noboru
39-15, Namiki-cho
Hachioji-shi Tokyo(JP)
Erfinder: Yamada, Takayo
2-16-2, Koyasu-machi
Hachioji-shi Tokyo(JP)
Erfinder: Wada, Katsuaki
39-15, Namiki-cho
Hachioji-shi Tokyo(JP)

(74) Vertreter: Schumacher, Günter, Dr. et al
c/o Bayer AG Konzernverwaltung RP
Patentabteilung
D-5090 Leverkusen 1 Bayerwerk(DE)

(54) Cyclische Carbamate.

(57) Neue cyclische Carbamate (I)

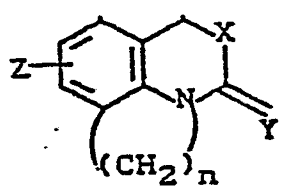

(I)

EP 0 239 927 A1

in der

X und Y jeweils Sauerstoff oder Schwefel bezeichnen,

Z Wasserstoff, Halogen, Alkyl oder Nitro bezeichnet, und

n 2 oder 3 ist sowie ihre Verwendung als Funigzide.

Die neuen Verbindungen der Formel (I) können z. B. hergestellt werden, indem man z. B. geeignete Hydroxymethylverbindungen mit Kohlenstoffdisulfid oder Carbonylsulfid umsetzt oder so erhaltene Verbindungen anschließend nitriert.

# Cyclische Carbamate

Die vorliegende Erfindung betrifft neue cyclische Carbamate, mehrere Verfahren zu ihrer Herstellung und ihre Verwendung als landwirtschaftliche Fungizide.

Es wurde bereits offenbart, daß bestimmte Benzoxazin-Derivate landwirtschaftlich-fungizide Aktivität haben (siehe die JP-OS 62829/1978) und daß bestimmte Benzazol-Derivate auch Aktivität als landwirtschaftliche Fungizide besitzen (siehe die JP-OS 132232/1979).

Neue cyclische Carbamate (I)

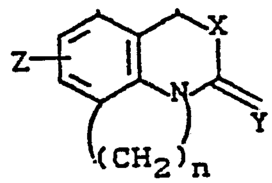

(I)

,

in der

X und Y jeweils Sauerstoff oder Schwefel bezeichnen,

Z Wasserstoff, Halogen, Alkyl oder Nitro bezeichnet, und

n 2 oder 3 ist,

wurden nunmehr gefunden.

Cyclische Carbamate der Formel (I) werden mittels eines Verfahrens erhalten, bei dem

(a) in dem Fall, in dem X und Y jeweils Sauerstoff bezeichnen und Z Wasserstoff, Halogen oder Alkyl bezeichnet, eine Verbindung der Formel (II)

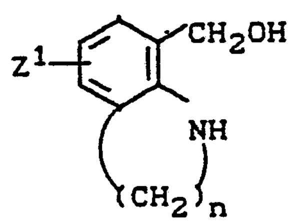

(II)

,

in der

n die im Vorstehenden angegebene Bedeutung hat und

Z' Wasserstoff, Halogen oder Alkyl bezeichnet, mit Trichloromethyl-chlorameisensäureester in Gegenwart eines inerten Lösungsmittels umgesetzt wird, oder

(b) in dem Fall, in dem X und Y jeweils Schwefel bezeichnen und Z die gleiche Bedeutung wie Z' hat, eine Verbindung der Formel (II) mit Kohlenstoffdisulfid in Gegenwart inerter Lösungsmittel, gegebenenfalls in Gegenwart von Alkalimetallhydroxid, umgesetzt wird, oder

(c) in dem Fall, in dem X Schwefel bezeichnet, Y Sauerstoff bezeichnet und Z die gleiche Bedeutung wie Z' hat, einer Verbindung der Formel (II) mit Carbonylsulfid in Gegenwart inerter Lösungsmittel, gegebenenfalls in Gegenwart von Alkalimetallhydroxid, umgesetzt wird, oder

(d) in dem Fall, in dem X und Y jeweils Sauerstoff bezeichen und Z Nitro bezeichnet, eine Verbindung der Formel (III)

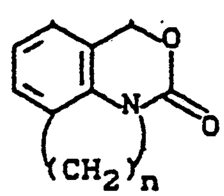

(III)

,

in der n die im Vorstehenden angegebenen Bedeutungen hat, in Gegenwart inerter Lösungsmittel mit Salpetersäure umgesetzt wird.

Die neuen cyclischen Carbamate zeigen potente Wirkung als landwirtschafliche Fungizide.

Überraschenderweise zeigen die erfindungsgemäßen cyclischen Carbamate eine wesentlich stärkere fungizide Wirkung auf landwirtschaftlischem Gebiet als die aus den vorerwähnten Druckschriften des Standes der Technik bekannten und, wie spezifisch aus den nachfolgenden biologischen Tests hervorgeht, haben die cyclischen Carbamate der Erfindung beispielsweise herausragende fungizide Wirkung gegen die Brusone-Krankheit von Reis (Pyricularia oryzae).

Von den erfindungsgemäßen cyclischen Carbamaten der Formel (I) sind bevorzugte Verbindungen diejenigen, in denen
X und Y jeweils Sauerstoff bezeichnen,
Z Wasserstoff, Fluor, Chlor, Brom, Nitro oder Alkyl mit 1 bis 4 Kohlenstoff-Atomen bezeichnet und
n 2 oder 3 ist.

Ganz besonders bevorzugte cyclische Carbamate der Formel (I) sind diejenigen, in denen
X und Y jeweils Sauerstoff bezeichnen,
Z Wasserstoff, Fluor, Chlor, Brom, Methyl oder Ethyl bezeichnet und
n 2 ist.

Speziell seien die folgenden Verbindungen erwähnt:
4,5-Dimethylen-2,4-benzoxazin-3-on und
7-Chloro-4,5-dimethylen-2,4-benzoxazin-3-on.

Wenn beispielsweise 7-Hydroxymethyl-2,3-dihydroindol und Trichloromethyl-chlorameisensäureester als Ausgangsstoffe in dem Verfahren (a) zur Herstellung der erfindungsgemäßen Verbindungen eingesetzt werden, läßt sich der Reaktionsablauf durch folgende Gleichung darstellen:

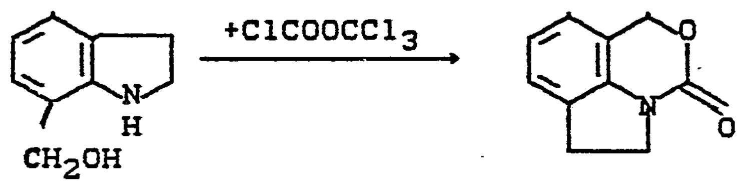


In gleicher Weise läßt sich, wenn 8-Hydroxymethyl-1,2,3,4-tetrahydrochinolin und Kohlenstoffdisulfid als Ausgangsstoffe in dem Verfahren (b) eingesetzt werden, der Reaktionsablauf durch die Gleichung darstellen:

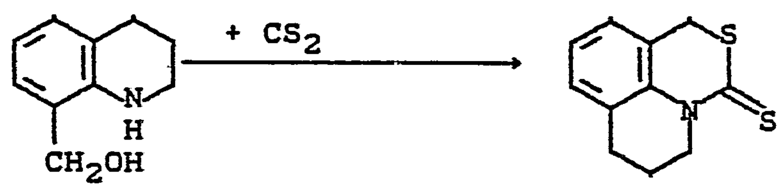


Wenn 8-Hydroxymethyl-1,2,3,4-tetrahydrochinolin und Carbonylsulfid als Ausgangsstoffe in dem Verfahren (c) eingesetzt werden, läßt sich der Reaktionsablauf durch die Gleichung darstellen:

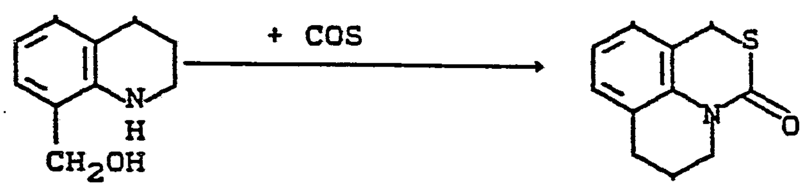


Wenn 4,5-Dimethylen-2,4-benzoxazin-3-on und Salpetersäure als Ausgangsstoffe in dem Verfahren (d) eingesetzt werden, läßt sich der Reaktionsablauf durch die Gleichung darstellen:

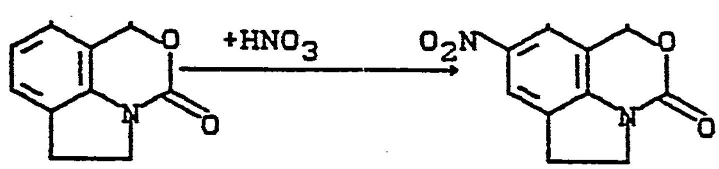

In den Verfahren (a), (b) und (c) haben die Verbindungen der Formel (II) die Bedeutungen, die auf den Definitionen von $Z^1$ und n beruhen. In der Formel (II) haben $Z^1$ und n vorzugsweise die bereits oben genannten Bedeutungen.

Zu den Verbindungen der Formel (II) zählen bestimmte neue Verbindungen. Beispiele für die neuen Verbindungen der Formel (II) sind folgende Verbindungen:
7-Hydroxymethyl-2,3-dihydroindol;
8-Hydroxymethyl-1,2,3,4-tetrahydrochinolin und
5-Chloro-7-hydroxymethyl-2,3-dihydroindol.

Das oben genannte 8-Hydroxymethyl-1,2,3,4-tetrahydrochinolin kann mittels eines Verfahrens hergestellt werden, das in J. Chem. Soc. Perkin Transactions, Vol. 2, No. 12, S. 1778 (1980) beschrieben wird. Die Verbindungen der Formel (II) können allgemein mittels eines Verfahrens hergestellt werden, bei dem eine Verbindung der Formel (IV)

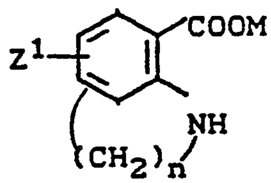

(IV)

,

in der $Z^1$ und n die oben angegebenen Bedeutungen haben und M Wasserstoff oder Niederalkyl bezeichnet, mit Lithiumaluminiumhydrid oder dergleichen reduziert wird.

Die Verbindungen der Formel (IV) umfassen bekannte Verbindungen, für die 1,2,3,4-Tetrahydrochinolin-3-carbonsäure, die in J. Chem. Soc., Vol. 68, S. 1844 (1946) offenbart ist, ein Beispiel ist. Die Verbindungen der Formel (IV) können allgemein in üblicher Weise dadurch hergestellt werden, daß eine bekannte Chinolin-8-carbonsäure [J. Chem. Soc., Vol. 68, S. 1844 (1946)] oder ein Indol-7-carbonsäuremethylester [JP-OS 14511/1976] oder ein Derivat desselben reduziert wird.

Die als Ausgangsstoffe bei dem Verfahren (d) eingesetzten Verbindungen der Formel (III) können mittels des Verfahrens (a) hergestellt werden, und sie sind Verbindungen der vorliegenden Erfindung.

Das Verfahren (a) wird vorzugsweise in Gegenwart eines geeigneten Lösungsmittels oder Verdünnungsmittels durchgeführt. Praktisch alle inerten organischen Lösungsmittel können zu diesem Zweck verwendet werden.

Beispiele für solche Lösungsmittel sind aliphatische, cycloaliphatische und aromatische, gegebenenfalls chlorierte Kohlenwasserstoffe wie Hexan, Cyclohexan, Petrolether, Ligroin, Benzol, Toluol, Xylol, Methylenchlorid, Chloroform, Kohlenstofftetrachlorid, Ethylen chlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; und Ester wie Ethylacetat; Amylacetat und dergleichen.

Die Reaktionstemperatur bei dem Verfahren (a) kann innerhalb eines weiten Bereichs variiert werden. Im allgemeinen kann das Verfahren (a) bei einer Temperatur von etwa 0°C bis 100°C, vorzugsweise bei einer Temperatur von etwa 50°C bis 80°C, durchgeführt werden. Die Reaktion läßt man vorzugsweise unter normalem Druck stattfinden, obwohl es auch möglich ist, die Reaktion bei erhöhtem oder vermindertem Druck durchzuführen.

Zur Durchführung des Verfahrens (a) können etwa 1 bis 1,5 mol, vorzugsweise etwa 1 bis 1,2 mol, Trichloromethylchlorameisensäureester auf 1 mol der Ausgangs-Verbindung (II) eingesetzt werden. Die Reaktion kann in einem inerten Lösungsmittel, beispielsweise in Ethylacetat, durchgeführt werden.

Bei der Durchführung des Verfahrens (b) kann man sich eines inerten Lösungsmittels bedienen, wie es in Verfahren (a) eingesetzt wird, wenngleich es auch möglich ist, andere Lösungsmittel wie Alkohole, Säureamide, Sulfone, Sulfoxide und dergleichen einzusetzen.

Die Reaktionstemperatur bei dem Verfahren (b) kann innerhalb eines weiten Bereichs variiert werden. Im allgemeinen kann die Reaktion bei einer Temperatur von etwa 0°C bis 60 °C, vorzugsweise bei einer Temperatur von etwa 20°C bis 40°C, durchgeführt werden. Obwohl die Reaktion vorzugsweise unter normalem Druck durchgeführt wird, ist es auch möglich, die Reaktion bei erhöhtem oder vermindertem Druck durchzuführen.

Bei der Durchführung des Verfahrens (b) kann Kohlenstoffdisulfid in einer Menge von etwa 1 bis 1,5 mol, vorzugsweise etwa 1 bis 1,2 mol, auf 1 mol der Ausgangs-Verbindung (II) eingesetzt werden. Die Reaktion kann in Gegenwart eines inerten Lösungsmittels, z.B. von Ethanol, und auch in Gegenwart von Natriumhydroxid durchgeführt werden.

Bei der Durchführung des Verfahrens (c) kann man sich eines inerten Lösungsmittels bedienen, wie es in Verfahren (a) eingesetzt wird.

Die Reaktionstemperatur bei dem Verfahren (c) kann innerhalb eines weiten Bereichs variiert werden. Im allgemeinen kann die Reaktion bei einer Temperatur von etwa -70°C bis +150 °C, vorzugsweise bei einer Temperatur von etwa -70°C bis +100°C, durchgeführt werden. Es ist vorteilhaft, die Reaktion unter normalem Druck durchzuführen, obwohl es auch möglich ist, einen höheren oder niedrigeren Druck anzuwenden.

Bei der Durchführung des Verfahrens (c) kann Carbonylsulfid in einer Menge von etwa 1 bis 1,5 mol, vorzugsweise etwa 1 bis 1,2 mol, auf 1 mol der Ausgangs-Verbindung (II) eingesetzt werden. Die Reaktion kann in Gegenwart eines inerten Lösungsmittels, z.B. von Ethanol, und auch in Gegenwart von Natriumhydroxid durchgeführt werden.

Bei der Durchführung des Verfahrens (d) kann man sich eines inerten Lösungsmittels bedienen, wie es in Verfahren (b) eingesetzt wird.

Die Reaktionstemperatur bei dem Verfahren (d) kann innerhalb eines weiten Bereichs variiert werden. Im allgemeinen kann die Reaktion bei einer Temperatur von etwa 0°C bis 40 °C, vorzugsweise bei einer Temperatur von etwa 0°C bis 20°C, durchgeführt werden.

Obwohl die Reaktion vorzugsweise unter normalem Druck durchgeführt wird, ist es auch möglich, die Reaktion bei höherem oder niedrigerem Druck durchzuführen.

Bei der Durchführung des Verfahrens (c) kann Salpetersäure in einer Menge von etwa 1 bis 1,5 mol, vorzugsweise etwa 1 bis 1,2 mol, auf 1 mol der Ausgangs-Verbindung (III) eingesetzt werden. Die Reaktion kann in Gegenwart eines inerten Lösungsmittels, z.B. einem Gemisch aus Eisessig und Essigsäureanhydrid, durchgeführt werden.

Die aktiven Verbindungen gemäß der Erfindung zeigen eine potente mikrobizide Wirkung und können in der Praxis zur Bekämpfung unerwünschter Mikroorganismen eingesetzt werden. Die aktiven Verbindungen sind zur Verwendung als Pflanzenschutzmittel geeignet.

Fungizide Mittel werden im Pflanzenschutz eingesetzt zur Bekämpfung von
Plasmodiophoromycetes,
Oomycetes,
Chytridiomycetes,
Zygomycetes,
Ascomycetes,
Basidiomycetes und
Deuteromycetes.

Bakterizide Mittel werden im Pflanzenschutz eingesetzt zur Bekämpfung von
Pseudomonodaceae,
Rhizobiaceae,
Enterobacteriaceae,
Corynebacteriaceae und
Streptomycetaceae.

Einige der Organismen, die pilzliche und bakterielle Erkrankungen verursachen und unter die oben aufgeführten Sammelbegriffe fallen, seien im Folgenden als nichtbeschränkende Beispiele genannt:
Species Xanthomonas
wie beispielsweise Xanthomonas campestris pv. oryzae;
Species Pseudomonas
wie beispielsweise Pseudomonas syringae pv. lacrymans;
Species Erwinia
wie beispielsweise Erwinia amylovora;
Species Pythium
wie beispielsweise Pythium ultimum;
Species Phytophthora
wie beispielsweise Phytophthora infestans;
Species Pseudoperonospora
wie beispielsweise Pseudoperonospora cubensis;
Species Plasmopara
wie beispielsweise Plasmopara viticola;
Species Peronospora
wie beispielsweise Peronospora pisi oder P. brassicae;
Species Erysiphe

wie beispielsweise Erysiphe graminis;
Species Sphaerotheca
wie beispielsweise Sphaerotheca fuliginea;
Species Podosphaera
wie beispielsweise Podosphaera leucotricha;
Species Venturia
wie beispielsweise Venturia inaequalis;
Species Pyrenophora
wie beispielsweise Pyrenophora teres oder P. graminea (Conidiale Form: Drechslera, Synonym: Helminthosporium);
Species Cochliobolus
wie beispielsweise Cochliobolus sativus (Conidiale Form: Drechslera, Synonym: Helminthosporium);
Species Uromyces
wie beispielsweise Uromyces appendiculatus
Species Puccinia
wie beispielsweise Puccinia recondita;
Species Tilletia
wie beispielsweise Tilletia caries;
Species Ustilago
wie beispielsweise Ustilago nuda oder Ustilago avenae;
Species Pellicularia
wie beispielsweise Pellicularia sasakii;
Species Piricularia
wie beispielsweise Piricularia oryzae;
Species Fusarium
wie beispielsweise Fusarium culmorum;;
Species Botrytis
wie beispielsweise Botrytis cinerea;
Species Septoria
wie beispielsweise Septoria nodorum;
Species Leptosphaeria
wie beispielsweise Leptosphaeria nodorum;
Species Cercospora
wie beispielsweise Cercospora canescens;
Species Alternaria
wie beispielsweise Alternaria brassicae;
Species Pseudocercosporella
wie beispielsweise Pseudocercosporella herpotrichoides.

Die gute Tolerierung der aktiven Verbindungen durch die Pflanzen bei den zur Bekämpfung der Pflanzenerkrankungen erforderlichen Konzentrationen erlaubt die Behandlung der oberirdischen Pflanzenteile, der Teile der vegetativen Fortpflanzung und der Samen sowie des Bodens.

Die aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche und synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe und ebenso auch Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischen Materialien wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat ebenso wie natürliche Phospholipide, etwa Cephaline und Lecithine, und synthetische Phospholipide können bei der Formulierung verwendet werden. Weitere Zusatzstoffe können mineralische und pflanzliche Öle sein.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-%, der aktiven Verbindung.

Die aktiven Verbindungen gemäß der vorliegenden Erfindung können in ihren Formulierungen oder ihren verschiedenen Anwendungsformen als Gemische mit anderen bekannten aktiven Verbindungen vorliegen, etwa mit Fungiziden, Bakteriziden, Insektiziden, Akariziden, Nematiziden, Herbiziden, vogelabweisenden Mitteln, Wachstumsfaktoren, Pflanzennährstoffen und Mitteln zur Verbesserung der Bodenstruktur.

Die aktiven Verbindungen können als solche eingesetzt werden, oder sie können in Form solcher Formulierungen oder Anwendungsformen, die daraus durch weitere Verdünnung hergestellt werden, etwa gebrauchsfertigen Lösungen, Emulsionen, Suspensionen, Pulvern, Pasten und Granulat, eingesetzt werden. Sie werden in üblicher Weise ausgebracht, etwa durch Bewässern, Eintauchen, Spritzen, Zerstäuben, Vernebeln, Verdampfen, Einspritzen, Bilden von Aufschlämmungen, Aufpinseln, Stäuben, Streuen, Trockenbedecken, Feuchtbedecken, Naßbedecken, Schlammbedecken und Umhüllen mit einer Kruste.

Bei der Behandlung von Pflanzenteilen können die Konzentrationen der aktiven Verbindung in den Anwendungsformen innerhalb eines beträchtlichen Bereichs variiert werden. Sie liegen im allgemeinen in einem Bereich von 1 bis 0,0001 Gew.-%, und vorzugsweise von 0,5 bis 0,001 Gew.-%.

Im Fall der Saatgut-Behandlung werden Mengen der aktiven Verbindung von beispielsweise 0,001 bis 50 g, vorzugsweise 0,01 bis 10 g, auf 1 kg des Saatguts allgemein zur Anwendung gebracht.

Im Fall der Behandlung des Bodens werden Konzentrationen der aktiven Verbindung am Ort der Wirkung von 0,00001 bis 0,1 Gew.-%, vorzugsweise 0,0001 bis 0,02 Gew.-%, allgemein zur Anwendung gebracht.

Die Erfindung wird durch die folgenden Beispiele näher erläutert. Es ist jedoch ausdrücklich anzumerken, daß der Umfang der Erfindung nicht auf die Beispiele beschränkt ist.

Herstellungsbeispiele

Beispiel 1

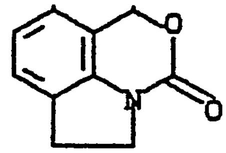

(Verbindung Nr. 1)

10 ml Trichloromethyl-chlorameisensäureester werden in 80 ml Ethylacetat gelöst. Zu der entstandenen Lösung wird eine Lösung von 4,00 g 7-Hydroxymethyl-2,3-dihydroindol in 20 ml Ethylacetat hinzugefügt.

Die Reaktionslösung wird 3 h unter Rückfluß erhitzt. Die Reaktionslösung wird auf Raumtemperatur abgekühlt, und das Lösungsmittel wird unter vermindertem Druck abdestilliert. Der Rückstand wird aus Ethanol umkristallisiert, wonach 3,34 g 4,5-Dimethylen-2,4-benzoxazin-3-on mit einem Schmp. 138-139 °C erhalten werden.

In analoger Weise wurden die folgenden Verbindungen hergestellt:

Verbindung Nr. 2

4,5-Trimethylen-2,4-benzoxazin-3-on
$^1$H-NMR ($CDCl_3$, $\delta$ ppm)
1,99 (2H, m), 2,77 (2H, t), 3,83 (2H, t), 5,17 (2H, s), 6,83 - 7,27 (3H, m).

Verbindung Nr. 3

6-Chloro-4,5-trimethylen-2,4-benzoxazin-3-on
$^1$H-NMR ($CDCl_3$, $\delta$ ppm)
2,00 (2H, m), 2,83 (2H, t), 3,73 (2H, t) 5,07 (2H, s), 6,80 (1H, d), 6,97 (1H, d).
Schmp. 98-103 °C.

Verbindung Nr. 4

7-Methyl-4,5-trimethylen-2,4-benzoxazin-3-on
$^1$H-NMR ($CDCl_3$, $\delta$ ppm)
2,07 (2H, m), 2,30 (3H, s), 2,73 (2H, t), 3,80 (2H, t), 5,13 (2H, s), 6,70 (1H, bs), 6,83 (1H, bs).
Schmp. 117-120 °C.

Verbindung Nr. 5

7-Chloro-4,5-trimethylen-2,4-benzoxazin-3-on
$^1$H-NMR ($CDCl_3$, $\delta$ ppm)
2,00 (2H, m), 2,70 (2H, t), 3,83 (2H, t), 5,10 (2H, s), 6,80 - 7,07 (2H, m).
Schmp. 106-111 °C.

Beispiel 2

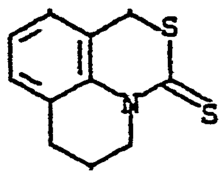

(Verbindung Nr. 6)

1,60 g 8-Hydroxymethyl-1,2,3,4-tetrahydrochinolin und 1,0 g Natriumhydroxid werden in 20 ml Ethanol gelöst, und 2 ml Kohlenstoffdisulfid werden dazugegeben. Die Reaktionslösung wird 12 h unter Rückfluß erhitzt. Die Reaktionsmischung wird auf Raumtemperatur abgekühlt. Das ausgefallene Rohprodukt wird durch Filtration abgetrennt und mit Ether gewaschen.
1,76 g 4,5-Trimethylen-2,4-benzothioxazin-3-thion werden als hellgelbliche Kristalle erhalten.
$^1$H-NMR ($CDCl_3$, δ ppm)
2,03 (2H, m), 2,83 (2H, t), 3,73 (2H, s), 4,47 (2H, t), 6,70 - 7,20 (3H, m).

In analoger Weise wie in Beispiel 2 wurden die folgenden Verbindungen hergestellt:

Verbindung Nr. 7

4,5-Dimethylen-2,4-benzothioxazin-3-thion
Schmp. 153-155 °C.

Verbindung Nr. 8

6-Chloro-4,5-trimethylen-2,4-benzothioxazin-3-thion
Schmp. 187-194 °C.

Verbindung Nr. 9

7-Methyl-4,5-trimethylen-2,4-benzothioxazin-3-thion
Schmp. <300 °C.

Beispiel 3

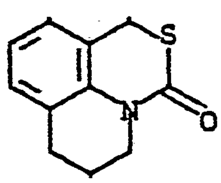

(Verbindung Nr. 10)

2,60 g 8-Hydroxymethyl-1,2,3,4-tetrahydrochinolin und 2,0 g Natriumhydroxid werden in 30 ml Ethanol gelöst. Die entstandene Lösung wird auf eine Temperatur von -70 °C gekühlt. Danach wird 1 h gasförmiges Carbonylsulfid bei -70°C in die Reaktionsmischung eingeleitet, so daß etwa 3 g Carbonylsulfid umgesetzt werden. Die Reaktionsmischung wird 4 h unter solchen Bedingungen gerührt, daß die Reaktionsmischung langsam auf Raumtemperatur erwärmt wird. Danach wird die Reaktionsmischung 3 h unter Rückfluß erhitzt.

Die Reaktionsmischung wird dann in 100 ml Wasser gegossen, und mit Hilfe von Ethylacetat wird eine Extraktion durchgeführt. Dann wird die Mischung über Natriumsulfat getrocknet. Das Lösungsmittel wird abdestilliert, wonach ein gelbes Öl erhalten wird, das dann der Chromatographie über eine Silicagel-Säule (Kohlenstofftetrachlorid/Chloroform = 1) unterworfen wird.
4,5-Trimethylen-2,4-benzothioxazin-3-on wird als gelbes Öl in einer Menge von 0,43 g erhalten.
$^1$H-NMR ($CDCl_3$, δ ppm)
1,93 (2H, m), 2,80 (2H, t), 3,30 (2H, t), 3,53 (2H, s), 6,37 - 7,10 (3H, m).

Beispiel 4

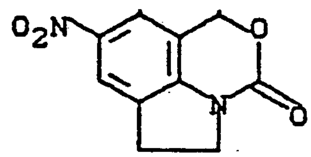

(Verbindung Nr. 11)

1,00 g 4,5-Dimethylen-2,4-benzoxazin-3-on wird in einer Mischung von 10 ml Eisessig 1,14 g Essigsäureanhydrid gelöst. Zu der entstandenen Lösung werden bei 10 °C unter Rühren 0,4 ml konzentrierte Salpetersäure hinzugefügt.

Die Reaktionslösung wird langsam auf Raumtemperatur erwärmt, und die Reaktionslösung wird weitere 3 h gerührt. Danach wird die Reaktionslösung in 50 ml Wasser gegossen und mit Ether extrahiert. Die organische Schicht wird mit Wasser gewaschen und über Natriumsulfat getrocknet.

Nach dem Abdestillieren des Lösungsmittels werden 0,47 g 7-Nitro-4,5-dimethylen-2,4-benzoxazin-3-on erhalten;
Schmp. 238-242 °C (Zers.).

Beispiel 5 (Herstellung der Zwischenprodukt-Verbindung)

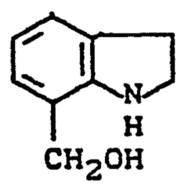

1,3 g Lithiumaluminiumhydrid werden in 10 ml wasserfreiem Ether unter einer Stickstoff-Atmosphäre suspendiert. Die erhaltene Suspension wird auf eine Temperatur von -20 °C gekühlt und mit einer Lösung von 2,00 g 2,3-Dihydroindol-7-carbonsäuremethylester in 20 ml wasserfreiem Ether versetzt. Die Reaktionslösung wird 1 h bei der oben erwähnten niedrigen Temperatur von -20 °C gerührt. Danach wird eine kleine Menge Wasser zu der Reaktionslösung gegeben, um das überschüssige Lithiumaluminiumhydrid zu hydrolysieren oder zu zersetzen.

Nachdem die Reaktionslösung sich auf Raumtemperatur erwärmt hat, wird die Reaktionslösung durch ein Celit-Filter filtriert. Das Lösungsmittel wird aus dem Filtrat abdestilliert, so daß 1,5 g 7-Hydroxymethyl-2,3-dihydroindol erhalten wurden.
$^1$H-NMR ($CDCl_3$, δ ppm)
2,93 (2H, t), 3,43 (2H, t), 4,43 (2H, t), 6,45 -6,93 (3H, m).

In analoger Weise wie in Beispiel 5 wurde die folgende Verbindung hergestellt:
5-Chloro-8-hydroxymethyl-1,2,3,4-tetrahydrochinolin
$^1$H-NMR ($CDCl_3$, $\delta$ ppm)
1,93 (2H, m), 2,70 (2H, t), 3,23 (2H, t), 4,16 (2H, s), 4,60 - 5,10 (2H, m), 6,47 (1H, d), 6,67 (1H, d).

Beispiel 6 (Herstellung von Ausgangsmaterial)

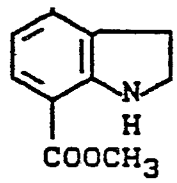

1,75 g Indol-7-carbonsäuremethylester und 2,92 g Boran-Trimethylamin-Komplex werden in 10 ml Dioxan gelöst. Zu der entstandenen Lösung werden 2 ml konzentrierte Salzsäure hinzugefügt. Die Reaktionslösung wird 30 min unter Rückfluß erhitzt und dann auf Raumtemperatur abgekühlt. 10 ml 6 n-Salzsäure werden zu der Reaktionsmischung hinzugefügt, die dann 15 min zum Rückfluß erhitzt und danach auf Raumtemperatur abgekühlt wird. Die Reaktionslösung wird in 50 ml Wasser gegossen. Die erhaltene Mischung wird mit wäßriger 2 n-Natriumhydroxid-Lösung neutralisiert und dann mit Ether extrahiert. Die organische Schicht wird mit einer gesättigten wäßrigen Natriumchlorid-Lösung gewaschen und über wasserfreiem Natriumsulfat getrocknet. Nach dem Abdestillieren des Lösungsmittels werden 1,26 g 2,3-Dihydroindol-7-carbonsäuremethylester erhalten; Schmp. 70-73 °C.

Biotest-Beispiele

Verwendungsbeispiele

Vergleichsverbindung A-1: (bekannt aus der JP-OS 62829-1978).

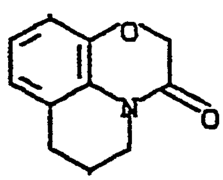

Vergleichsverbindung B-1 (bekannt aus der JP-OS 132232-1979).

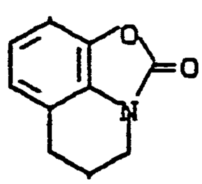

Verwendungsbeispiele

Test gegen die Brusone-Krankheit bei Reispflanzen -Aufbringen der aktiven Verbindungen auf Stengel und Blätter der Pflanzen.

Formulierung der aktiven Verbindung

Aktive Verbindung: 50 Gew.-Teile
Träger: Mischung aus Diatomeenerde und Kaolin (1:5) 45 Gew.-Teile
Emulgator: Polyoxyethylenalkylphenylether 5 Gew.-Teile

Die angegebene Menge der aktiven Verbindung wurde mit der angegebenen Menge Träger und der angegebene Menge Emulgator vermahlen und vermischt, wodurch eine konzentrierte, emulgierbare Zusammensetzung erhalten wurde, die danach mit Wasser verdünnt wurde.

Biotest-Beispiel A

Test-Verfahren

Reispflanzen (Varietät Kusabue) wurden in Töpfen aus Vinyl-Kunststoff mit einem Durchmesser von 7,5 cm gezoden. Sobald die Pflanzen bis zum 3-oder 4-Blatt-Stadium gewachsen waren, wurden sie mit einer Sprühlösung besprüht, die die aktive Verbindung in einer vorher festgelegten Konzentration enthielt. Die Menge der Sprühlösung betrug 50 ml auf jeweils 3 Töpfe.

Am nächsten Tag wurden die Pflanzen mittels eines Sprüh-Arbeitsganges mit einer wäßrigen Sporen-Suspension des Brusone-Pilzes inokuliert und in einer Feuchtigkeitskammer bei 25 °C und einer relativen Luftfeuchtigkeit von 100 % inkubiert.

7 Tage nach dem Inokulieren wurde der Infektionsgrad der Reispflanzen mittels der folgenden Skala bestimmt.

| Grad der Infektion | Infizierte Fläche (%) bezogen auf die Gesamtfläche der beobachteten Pflanzenteile |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

Der Grad des Pflanzenschutzes (%) wurde nach der nachstehenden mathematischen Formel berechnet:

$$\text{Grad des Pflanzenschutzes (\%)} = \left[\frac{X - Y}{X}\right] \times 100 ,$$

worin
X = Grad der Infektion in unbehandelten Pflanzen (Kontrolle);
Y = Grad der Infektion in behandelten Pflanzen.

In diesem Test bestand eine Behandlung aus drei Töpfen. Die Ergebnisse sind in Tabelle A aufgeführt.

## Tabelle A

| Aktive Verbindung Nr. | Menge der aktiven Verbindung (ppm) | Grad der Schutzes (%) |
|---|---|---|
| 1 | 100 | 100 |
| 2 | 100 | 100 |
| 3 | 100 | 100 |
| 4 | 100 | 100 |
| 6 | 100 | 100 |
| Vergleichsverbindung | | |
| A-1 | 100 | 80 |
| B-1 | 100 | 74 |

Biotest-Beispiel B

Test gegen die Brusone-Krankheit bei Reispflanzen auf bewässerten Reisfeldern -Aufbringen der aktiven Verbindungen auf die Wasseroberfläche des bewässerten Reisfeldes

Test-Verfahren

Reispflanzen (Varietät Kusabue) wurden in weißben Porzellantöpfen mittels Bewässerung mit Wasser gezogen, mit der Maßgabe, daß die Zahl der Pflanzen pro Topf 3 betrug.

Sobald die Reispflanzen bis zum Stadium der frühen Bodenbearbeitung oder dem Stadium der Bildung der jungen Ähren gewachsen waren, wurde eine vorher festgelegte Menge einer fungiziden Lösung in das Oberflächenwasser der Töpfe gegossen.

Die fungizide Lösung wurde in der gleichen Weise wie in Biotest-Beispiel A hergestellt und enthielt die aktive Verbindung in einer vorher festgelegten Konzentration.

Nach 21 Tagen wurden die Reispflanzen in üblicher Weise mittels eines Sprüh-Arbeitsganges mit einer wäßrigen Sporen-Suspension des Brusone-Pilzes inokuliert. Die Pflanzen wurden dann 24 h in einen Raum mit 25 °C und einer relativen Luftfeuchtigkeit von 100 % gestellt.

Danach wurden die Pflanzen in einem Gewächshaus bei einer Temperatur von 20 °C bis 28 °C gehalten.

Sieben Tage nach der Inokulierung wurden die Reispflanzen beobachtet, und der Grad des Schutzes (%) wurde wie in Biotest-Beispiel A berechnet.

Die Ergebnisse sind in Tabelle B aufgeführt.

Tabelle B

| Aktive Verbindung Nr. | Menge der aktiven Verbindung ($mg/m^2$) | Grad der Schutzes (%) |
|---|---|---|
| 1 | 200 | 100 |
| 2 | 200 | 100 |
| 3 | 200 | 100 |
| 4 | 200 | 100 |
| Vergleichsverbindung | | |
| A-1 | 200 | 60 |
| B-1 | 200 | 47 |

**Ansprüche**

1. Cyclisches Carbamat der Formel (I)

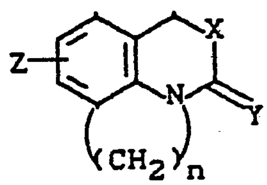

(I) ,

in der
X und Y jeweils Sauerstoff oder Schwefel bezeichnen,
Z Wasserstoff, Halogen, Alkyl oder Nitro bezeichnet, und
n 2 oder 3 ist.

2. Verbindungen der Formel (I) nach Anspruch 1, dadurch gekennzeichnet, daß
X und Y jeweils Sauerstoff bezeichnen,
Z Wasserstoff, Fluor, Chlor, Brom, Nitro oder Alkyl mit 1 bis 4 Kohlenstoff-Atomen bezeichnet und
n 2 oder 3 ist.

3. Verbindungen der Formel (I) nach Anspruch 1, dadurch gekennzeichnet, daß
X und Y jeweils Sauerstoff bezeichnen,
Z Wasserstoff, Fluor, Chlor, Brom, Methyl oder Ethyl bezeichnet und
n 2 ist.

4. 4,5-Dimethylen-2,4-benzoxazin-3-on nach Ansprüchen 1 bis 3, bezeichnet durch die Formel

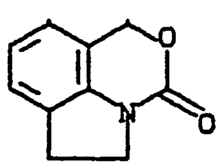

.

5. Verfahren zur Herstellung cyclischer Carbamate der Formel (I)

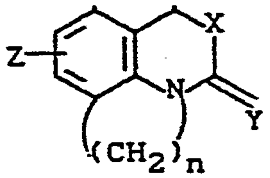

(I)

,

in der
X und Y jeweils Sauerstoff oder Schwefel bezeichnen,
Z Wasserstoff, Halogen, Alkyl oder Nitro bezeichnet, und
n 2 oder 3 ist,
dadurch gekennzeichnet, daß

(a) in dem Fall, in dem X und Y jeweils Sauerstoff bezeichnen und Z Wasserstoff, Halogen oder Alkyl bezeichnet, eine Verbindung der Formel (II)

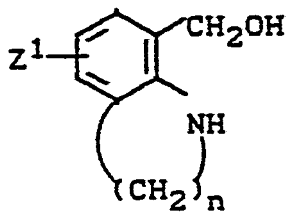

(II)

,

in der
n die im Vorstehenden angegebene Bedeutung hat und
$Z^1$ Wasserstoff, Halogen oder Alkyl bezeichnet, mit Trichloromethyl-chlorameisensäureester in Gegenwart eines inerten Lösungsmittels umgesetzt wird, oder

(b) in dem Fall, in dem X und Y jeweils Schwefel bezeichnen und Z die gleiche Bedeutung wie $Z^1$ hat, eine Verbindung der Formel (II) mit Kohlenstoffdisulfid in Gegenwart inerter Lösungsmittel, gegebenenfalls in Gegenwart von Alkalimetallhydroxid, umgesetzt wird, oder

(c) in dem Fall, in dem X Schwefel bezeichnet, Y Sauerstoff bezeichnet und Z die gleiche Bedeutung wie $Z^1$ hat, einer Verbindung der Formel (II) mit Carbonylsulfid in Gegenwart inerter Lösungsmittel, gegebenenfalls in Gegenwart von Alkalimetallhydroxid, umgesetzt wird, oder

(d) in dem Fall, in dem X und Y jeweils Sauerstoff bezeichnen und Z Nitro bezeichnet, eine Verbindung der Formel (III)

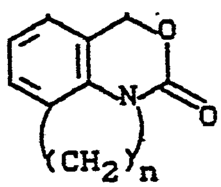

(III)

,

in der n die im Vorstehenden angegebenen Bedeutungen hat, in Gegenwart inerter Lösungsmittel mit Salpetersäure umgesetzt wird.

6. Fungizide Mittel für Landwirtschaft und Gartenbau, dadurch gekennzeichnet, daß sie wenigstens ein cyclisches Carbamat der Formel (I) enthalten.

7. Verfahren zur Bekämpfung von Pflanzenkrankheiten, dadurch gekennzeichnet, daß man cyclische Carbamate der Formel (I) auf die Pathogene und/oder den Ort ihres Auftretens oder den Ort des Auftretens der Pflanzenkrankheit einwirken läßt.

8. Verwendung von cyclischen Carbamaten der Formel (I) zur Bekämpfung von Pflanzenkrankheiten.

9. Verfahren zur Herstellung von fungiziden Mittel für Landwirtschaft und Gartenbau, dadurch gekennzeichnet, daß cyclische Carbamate der Formel (I) mit Streckmitteln und/oder grenzflächenaktiven Mitteln vermischt werden.

Europäisches Patentamt

# EUROPÄISCHER RECHERCHENBERICHT

Nummer der Anmeldung

EP 87 10 4430

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 103 797 (BAYER)<br>* Anspruch 1; Seite 7, Zeilen 4-6 * | 1,6 | C 07 D 498/06<br>C 07 D 513/06<br>A 01 N 43/90 //<br>(C 07 D 498/06<br>C 07 D 265:00<br>C 07 D 209:00 )<br>(C 07 D 498/06<br>C 07 D 265:00<br>C 07 D 221:00 )<br>(C 07 D 513/06<br>C 07 D 279:00<br>C 07 D 209:00 )<br>(C 07 D 513/06<br>C 07 D 279:00<br>C 07 D 221:00 ) |
| Y | PATENT ABSTRACTS OF JAPAN, Band 4, Nr. 173 (C-32)[655], 29. November 1980; & JP-A-55 111 406 (TAKEDA YAKUHIN KOGYO K.K.) 28.08.1980<br>* Zusammenfassung * | 1,8 | |
| | | | **RECHERCHIERTE SACHGEBIETE (Int. Cl.4)** |
| | | | C 07 D 498/00<br>C 07 D 513/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 28-06-1987 | SCARPONI U. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82